# EUROPEAN PATENT SPECIFICATION

(11) **EP 1 578 738 B1**
(45) Date of publication and mention of the grant of the patent: **12.03.2008**
(21) Application number: 03779759.4
(22) Date of filing: 18.12.2003
(51) Int. Cl.: C07D 307/87, A61K 31/343, A61P 25/00, A61P 25/22, A61P 25/24, A61P 25/30

(54) **ESCITALOPRAM HYDROBROMIDE AND A METHOD FOR THE PREPARATION THEREOF**
ESCITALOPRAMHYDROBROMID UND EIN VERFAHREN ZU DESSEN HERSTELLUNG
HYDROBROMURE D'ESCITALOPRAM ET SON PROCEDE DE PREPARATION

(30) Priority: 23.12.2002 DK 200202005
(43) Date of publication of application: 28.09.2005
(73) Proprietor: H. LUNDBECK A/S, 2500 Valby-Copenhagen (DK)
(72) Inventor: MARTEL, Lawrence, Manchester, NH 03104 (US); DANCER, Robert, DK-2650 Hvidovre (DK); PETERSEN, Hans, DK-2720 Vanl se (DK); ELLEGAARD, Peter, DK-4174 Jystrup (DK)
(86) International application number: PCT/DK2003/000902
(87) International publication number: WO 2004/056791

(56) References cited:
- EP-A1- 0 347 066
- EP-A1- 1 152 000
- EP-B- 0 171 943
- WO-A-03/000672
- WO-A1-01/03694
- WO-A1-02/087566

## Description

The present invention relates to escitalopram, which is the S-enantiomer of the well-known antidepressant drug citalopram, i.e. (S)-1-[3-(dimethylanfno)propyl]-1-(4-fluorophenyl)-1,3-dihydro-5-isobenzofurancarbonitrile, in the form of its hydrobromide as well as a method for the preparation thereof.

### Background of the Invention

Citalopram is a well-known antidepressant drug that has now been on the market for some years and has the following structure:

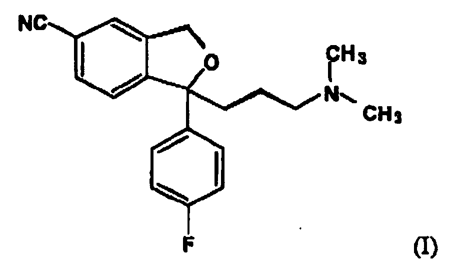

It is a selective, centrally acting serotonin (5-hydroxytryptamine; 5-HT) reuptake inhibitor, accordingly having antidepressant activities. Citalopram was first disclosed in DE 2,657,013, corresponding to US 4,136,193.

The diol, 4-[4-(dimethylamino)-1-(4'-fluorophenyl)-1-hydroxy-1-butyl]-3-(hydroxy methyl)-benzanitrile, and its use as an intermediate in the preparation of citalopram has been disclosed in e.g. US patent No 4,650,884.

European Patent Application No 1152000 discloses citalopram hydrobromide crystals containing crystals having a particle size of less than 5 µm in a proportion of 35% at most as well as methods for preparing such crystals.

The S-enantiomer (escitalopram) of the formula 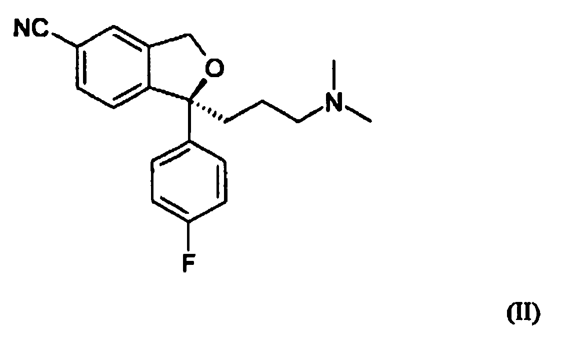 and the antidepressant effect of said enantiomer is disclosed in US Patent No 4,943,590. EP patent application No. 1.200.081 describes the use of escitalopram for the treatment of neurotic disorders and WO 02/087566 describes the use of escitalopram for treating depressive patients who have failed to respond to conventional SSRIs and for treating a number of other disorders.

Methods for the preparation of escitalopram are disclosed in US Patent No 4,943,590 and a number of other patent applications.

This application also describes the free base of escitalopram as an oil, the oxalic acid salt, the pamoic acid and the L-(+)-tartaric acid addition salt of escitalopram. Due to the toxicity of pamoic acid addition salts they are not suitable in pharmaceuticals.

Escitalopram has now been developed as an antidepressant and a need for alternative salts of escitalopram has emerged.

In the search for salts suitable for a pharmaceutical composition more than 30 organic and inorganic acids were investigated in different solvent systems and under different conditions. These acids gave either oils or amorphous solids having moderate to high hygroscopic properties. No non-hydroscopic crystalline solids were formed from monocarboxylic organic acids and these acids formed mostly oils. Di- and triphasic organic acids gave amorphous solids. Interestingly, the salt formed with L-tartaric acid was an amorphous solid.

Thus, very few crystalline, stable, non-hygroscopic salts of escitalopram are known.

Formation of crystalline salts with hydrochloric acid and hydrobromic acids have until now been unsuccessful.

It has now been found that crystalline escitalopram hydrobromide may be formed using gaseous hydrobromide under anhydrous conditions.

### Summary of the invention

The present invention relates to escitalopram hydrobromide in crystalline form.

The invention also relates to a pharmaceutical composition containing escitalopram hydrobromide and one or more pharmaceutically acceptable carriers or diluents.

Finally the present invention relates to the use of escitalopram hydrobromide according to claims 1 for the manufacture of a pharmaceutical composition for the treatment of depression including treatment of patients which have failed to respond to initial treatment with conventional SSRIs, neurotic disorders (such as generalized anxiety disorder, social anxiety disorder, obsessive compulsive disorder, post traumatic stress disorder and panic attacks including panic disorder, social phobia, specific phobias and agoraphabia), acute stress disorder, eating disorders such as bulimia, anorexia and obesity, dysthymia, pre-menstrual syndrome, cognitive disorders, impulse control disorders, attention deficit hyperactivity disorder and drug abuse.

### Detailed description of the invention

It has been found that salt formation is very sensitive to the presence of water and salt formation should therefore be carried out under anhydrous conditions. Preferably salt formation is carried out by dissolving escitalopram in an anhydrous solvent, such as acetone or a ketone with a larger molecule weight, such as methyl-isobutylketone. Preferably the anhydrous solvent is one that does not easily pick up water. The hydrobromic acid is suitably added as a gas.

Another method for making crystalline escitalopram hydrobromide comprises preparing an anhydrous solution or almost anhydrous solution of hydrogen bromide in an organic solvent (such as *iso*-propyl alcohol) by bubbling anhydrous hydrogen bromide gas through the organic solvent. A suitable aliquot of this solution is then added to a solution of escitalopram base in an organic solvent.

A third method for making crystalline escitalopram hydrobromide comprises adding an aqueous solution of hydrobromic acid to escitalopram free base to form the salt, with the water being removed subsequently by means known to the skilled chemist (for example drying by azeotropic distillation using for example toluene or *iso*-propyl alcohol, or drying using a solid drying agent). The escitalopram free base may be in solid form, an oil or a solution.

### Examples

A) Experiment with HCl gas: A 250 ml round bottom flask was charged with 5.7 g escitalopram free base and 120 ml isopropanol. The mixture was stirred until a homogenous solution was obtained. The mixture was cooled to 5 °C and HCL gas was bubbled in for 20 minutes with cooling. The mixture was placed in the refrigerator overnight. No solid material was formed. The mixture was then concentrated in vacuo to an oil and the oily residue was dissolved in acetone by heating to 45 °C (the solution was a 0.5 molar solution in acetone). The flask was scratched to initiate nucleation and the solution became cloudy. The solution was cooled to 5 °C overnight. The fluffy material was collected by filtration and transferred to an amber bottle for drying (50 °C HI-VAC) to a powder. A sample of this powder was exposed to air and as it picked up water from the air is became an oil. Attempts were made to recrystallise the oil using a variety of different solvents. None of these trials resulted in a solid product.
B) Experiment with HBr gas: This experiment was run exactly as the experiment above except HBr gas was bubbled into the solution in *iso*-propanol. No solid material was formed in *iso*-propanol but on solvent change to acetone (a 0.5 molar solution) an off-white solid formed. The solid was collected, washed with cold acetone to give a crystalline material. The crystalline escitalopram hydrobromide was found by melting point, HPLC, and proton NMR to have a good purity. A sample of the material was exposed to air and it was found to be non-hygroscopic.
C) Experiments with different solvents: These experiments were performed as follows: To a solution of the escitalopram free base (approx. 20 % w/w) in dry 2-propanol was added dropwise 0,9-1,0 eq. of HBr (g) dissolved in dry 2-propanol. Precipitation of a solid normally occurred within 30 minutes. Where the precipitation was performed in a solvent other than 2-propanol, the resulting mixture was evaporated under reduced pressure and the appropriate solvent was added, evaporated again and the appropriate solvent given one more time to the mixture before final crystallisation.

Below is a table showing results from different solvents:

### Precipitation of escitalopram hydrobromide from different solvents

| Solvent | Yield | Purity (HPLC) | Melting Point |
|---|---|---|---|
| Toluene | 81 % | 99,1 % | 131 °C |
| MTBE/IPA (200:55) | 72 % | 98,3 % | 132 °C |
| IPA | 67 % | 99,4 % | 133 °C |
| MTBE | 93,4 % | 99,2 % | 131,6 °C |
| THF | 54,5 % | 99,95 % | 133,9 °C |
| Butanone | 30 % | 100 % | 133-134 °C |
| *n*-Butanol | 67 % | 99,9 % | 133-134 °C |
| *iso*-Butanol | 66 % | 99,6 % | 133-134 °C |
| *tert*-Butanol/IPA (4:1) | 82 % | 99,9 % | 133-134 °C |
| 2-Butanol | 85 % | 100 % | 133-134 °C |
| MIBK | 75 % | 100 % | - |
| 2-methyl-THF | 84 % | 100 % | - |
| 1,4-Dioxane | 65 % | 100 % | - |
| Ether | 91 % | 100 % | - |
| EtOAc | 88 % | 100 % | - |

| | | | |
|---|---|---|---|
| MTBE = methyl *t*-butyl ether; IPA = *iso*-propanol; MIBK = methyl *iso*-butyl ketone; THF = tetrahydrofuran; EtOAc = ethyl acetate. | | | |

Other solvents, such as acetonitrile, methanol, ethanol and propylencarbonate, were tried, but gave no crystallisation:

Another approach is to dissolve the base in 2-propanol, add 0,9 - 1,0 eq. of aqueous hydrobromic acid, distil off the solvent and remove the water by repeated azeotropic distillations (e.g. 2-propanol and toluene). This procedure seems to give somewhat lower yields.

## Claims

1. Escitalopram hydrobromide in crystalline form.

2. A pharmaceutical composition containing escitalopram hydrobromide according to claim 1 and pharmaceutically acceptable carriers and diluents.

3. The use of escitalopram hydrobromide according to claim 1 for the manufacture of a pharmaceutical composition for the treatment of depression including treatment of patients which have failed to respond to initial treatment with conventional SSRIs, neurotic disorders (such as generalized anxiety disorder, social anxiety disorder, obsessive compulsive disorder, post traumatic stress disorder and panic attacks including panic disorder, social phobia, specific phobias and agoraphobia), acute stress disorder, eating disorders such as bulimia, anorexia and obesity, dysthymia, pre-menstrual syndrome, cognitive disorders, impulse control disorders, attention deficit hyperactivity disorder and drug abuse.

## Patentansprüche

1. Escitalopramhydrobromid in kristalliner Form.

2. Pharmazeutische Zusammensetzung, die Escitalopramhydrobromid gemäß Anspruch 1 und pharmazeutisch akzeptable Träger und Verdünnungsmittel enthält.

3. Verwendung von Escitalopramhydrobromid gemäß Anspruch 1 zur Herstellung einer pharmazeutischen Zusammensetzung zur Behandlung von Depression, einschließend die Behandlung von Patienten, die auf die anfängliche Behandlung mit herkömmlichen SSRIs nicht angesprochen haben, neurotischen Störungen (wie allgemeine Angststörung, soziale Angststörung, Zwangsneurose, posttraumatische Stressstörung und Panikattacken, die Panikstörung, soziale Phobie, spezifische Phobien und Agoraphobie einschließen), akuter Stressstörung, Essstörungen, wie Bulimie, Anorexie und Fettsucht, Dysthymie, prämenstruellem Syndrom, kognitiven Störungen, Impulskontrollstörungen, hyperaktiver Störung mit Aufmerksamkeitsdefizit und Drogenmissbrauch.

## Revendications

1. Bromhydrate d'escitalopram sous forme cristalline.

2. Composition pharmaceutique contenant du bromhydrate d'escitalopram selon la revendication 1 et des supports et diluants pharmaceutiquement acceptables.

3. Utilisation de bromhydrate d'escitalopram selon la revendication 1 pour la fabrication d'une composition pharmaceutique destinée au traitement de la dépression, comprenant le traitement de patients n'ayant pas répondu au traitement initial avec des IRSS classiques, les troubles névrotiques (comme l'anxiété généralisée, l'anxiété sociale, les troubles obsessionnels compulsifs, le stress post-traumatique et les attaques de panique, y compris le trouble de panique, la phobie sociale, les phobies spécifiques et l'agoraphobie), le stress aigu, les troubles de l'alimentation comme la boulimie, l'anorexie et l'obésité, l'humeur dépressive, le syndrome prémenstruel, les troubles cognitifs, les troubles du contrôle des impulsions, les troubles d'hyperactivité avec déficit de l'attention et la toxicomanie.
